# EUROPEAN PATENT APPLICATION

(11) **EP 4 461 347 A1**
(43) Date of publication of application: **13.11.2024**
(21) Application number: 24163042.5
(22) Date of filing: 12.03.2024
(51) Int. Cl.: A61N 5/10

(54) **METHODS, APPARATUS AND COMPUTER-READABLE MEDIA FOR MANAGING RADIOTHERAPY**

(30) Priority: 11.05.2023 GB 202306997
(71) Applicant: Elekta Limited, West Sussex RH10 9RR (GB)
(72) Inventor: HOLMES, Kathrin, Crawley, RH10 9RR (GB); CLARK, Gary, Crawley, RH10 9RR (GB)
(74) Representative: Haseltine Lake Kempner LLP

(57) **Abstract**

A method is disclosed, performed by a management apparatus for a radiotherapy system, the radiotherapy system comprising a source of therapeutic radiation mounted on a rotatable gantry. The method comprises: receiving, from a treatment planning apparatus, a treatment plan for delivery by the radiotherapy system, the treatment plan specifying a plurality of beams to be generated by the source of therapeutic radiation, wherein each beam is associated with first and second angles of the rotatable gantry, wherein the beam is initiated at one of the first and second angles, and terminated at the other of the first and second angles; applying an optimization algorithm to the plurality of beams, the optimization algorithm arranging the plurality of beams into an order such that an aggregate amount of rotation by the rotatable gantry between beams when delivering the treatment plan is minimized; and outputting the ordered plurality of beams to the radiotherapy system for implementation.

## Description

### Technical field

Embodiments of the disclosure relate to radiotherapy, and particularly to methods, apparatus and computer-readable media for managing radiotherapy.

### Background

Radiotherapy is used to treat cancers and other ailments in mammalian (e.g., human and animal) tissue. In such treatment, cells forming part of the tumour are irradiated by ionising radiation (e.g., high-energy x-rays, electrons, alpha particles, etc) in order to destroy or damage them. In order to apply a prescribed dose of ionising radiation to a target location or target region, such as a tumour, the ionising radiation will typically also pass through healthy tissue of the human or animal body. Therefore, while radiotherapy has the desirable consequence of irradiating and damaging a target region, it can also have the undesirable consequence of irradiating and damaging healthy tissue.

Modern radiotherapy treatment uses techniques to reduce the radiation dose to healthy tissue and thereby provide a safe treatment. For example, one approach to minimising a radiation dose received by healthy tissue surrounding a target region is to direct the radiation towards the target region from a plurality of different angles, for example by rotating a source of radiation around the patient by use of a rotating gantry. In such modes of treatment, a plurality of beams are defined, each directed at the target region but generated from a different angle of rotation of the gantry. Radiative dose thus accumulates in the target region, but does not accumulate to such an extent in the surrounding healthy tissue. The direction and shape of each radiation beam should be accurately controlled to ensure the tumour receives the prescribed radiation, and the placement of the beams should be such as to minimize dose to the surrounding healthy tissue and especially critical organs.

Treatment planning can be used to control radiation beam parameters. Traditionally, for each patient, a radiation therapy treatment plan ("treatment plan") may be created using an optimization technique based on clinical and dosimetric objectives and constraints. For example, an oncologist may define a minimum dose that is to be applied to the target region (i.e., "at least dose X applied to the target region"), and a maximum dose that is to be applied to healthy critical organs and other surrounding tissue (i.e., "no more than dose *Y* to that critical organ"). Another dosimetric objective may be generally to minimize the dose to healthy tissue. An optimization algorithm is then run on a three-dimensional image of the target region and its surrounding area to find a set of radiation beam parameters that achieves the dosimetric and clinical objectives.

The output of the optimization algorithm, the treatment plan, defines one or more radiation beams and their associated parameters. For example, each radiation beam may be defined by one or more of the following parameters (among others):
- A start angle of the rotatable gantry, at which the radiation beam is initiated.
- A stop angle of the rotatable gantry, at which the radiation beam is halted.
- A cross-sectional shape of the radiation beam, as defined by one or more collimating elements.
- An energy of the radiation beam.

Note that the start angle and the stop angle for a particular radiation beam may be the same or different. In the former case, the radiation beam may be known as "stop and shoot", i.e., the radiation beam is delivered while the gantry is stationary, from a single angle. In the latter case, the radiation beam is delivered while the gantry rotates from the start angle to the stop angle, defining an arc. In this case, the cross-sectional shape of the radiation beam can be the same throughout the arc, or it may change as the gantry rotates, e.g., to correspond to the cross-section of the target region from the different angles.

The treatment plan is output to a radiotherapy system for delivery to the patient. Typically, each treatment plan will define multiple beams, delivered from different angles of rotation of the gantry. These beams are delivered sequentially in the order defined by the treatment plan.

### Summary

One key performance indicator for radiotherapy systems and radiotherapy treatment in general is the number of patients that can be treated effectively in a given period of time. There are two complementary aspects to this efficacy. One is the efficacy of the treatment itself. If radiotherapy is 100% successful in all cases with zero negative side-effects, clearly that is a powerful factor in support of the use of such therapy. The other aspect is the amount of time that each treatment takes. If a given treatment can be completed relatively quickly, more patients can be treated and receive the benefits of radiotherapy. Embodiments of the present disclosure seek to address this latter aspect, and particularly provide methods, systems and computer-readable media which reduce the amount of time taken to deliver a given treatment to a patient.

According to a first aspect, there is provided method, performed by a management apparatus for a radiotherapy system, the radiotherapy system comprising a source of therapeutic radiation mounted on a rotatable gantry. The method comprises: receiving, from a treatment planning apparatus, a treatment plan for delivery by the radiotherapy system, the treatment plan specifying a plurality of beams to be generated by the source of therapeutic radiation, wherein each beam is associated with first and second angles of the rotatable gantry, wherein the beam is initiated at one of the first and second angles, and terminated at the other of the first and second angles; applying an optimization algorithm to the plurality of beams, the optimization algorithm arranging the plurality of beams into an order such that an aggregate amount of rotation by the rotatable gantry between beams when delivering the treatment plan is minimized; and outputting the ordered plurality of beams to the radiotherapy system for implementation.

Apparatus for performing the method according to the first aspect is also provided. For example, the disclosure provides a management apparatus configured to perform the method according to the first aspect. In another example, the management apparatus comprises processing circuitry and a non-transitory computer-readable medium storing instructions which, when executed by the processing circuitry, cause the management apparatus to: receive, from a treatment planning apparatus, a treatment plan for delivery by a radiotherapy system comprising a source of therapeutic radiation mounted on a rotatable gantry, the treatment plan specifying a plurality of beams to be generated by the source of therapeutic radiation, wherein each beam is associated with first and second angles of the rotatable gantry, wherein the beam is initiated at one of the first and second angles, and terminated at the other of the first and second angles; apply an optimization algorithm to the plurality of beams, the optimization algorithm arranging the plurality of beams into an order such that an aggregate amount of rotation by the rotatable gantry between beams when delivering the treatment plan is minimized; and output the ordered plurality of beams to the radiotherapy system for implementation.

In another aspect, a non-transitory computer-readable medium stores instructions which, when executed by processing circuitry, cause the processing circuitry to: receive, from a treatment planning apparatus, a treatment plan for delivery by a radiotherapy system comprising a source of therapeutic radiation mounted on a rotatable gantry, the treatment plan specifying a plurality of beams to be generated by the source of therapeutic radiation, wherein each beam is associated with first and second angles of the rotatable gantry, wherein the beam is initiated at one of the first and second angles, and terminated at the other of the first and second angles; apply an optimization algorithm to the plurality of beams, the optimization algorithm arranging the plurality of beams into an order such that an aggregate amount of rotation by the rotatable gantry between beams when delivering the treatment plan is minimized; and output the ordered plurality of beams to the radiotherapy system for implementation.

### Brief description of the drawings

Exemplary embodiments will now be described, by way of example only, with reference to the following drawings, in which:
Figure 1 is a schematic diagram of a radiotherapy apparatus;
Figure 2 is a flowchart of a method according to embodiments of the disclosure;
Figure 3 is a flowchart of a method according to embodiments of the disclosure; and
Figure 4 is a schematic illustration of a computer-readable medium according to embodiments of the present disclosure.

### Detailed description

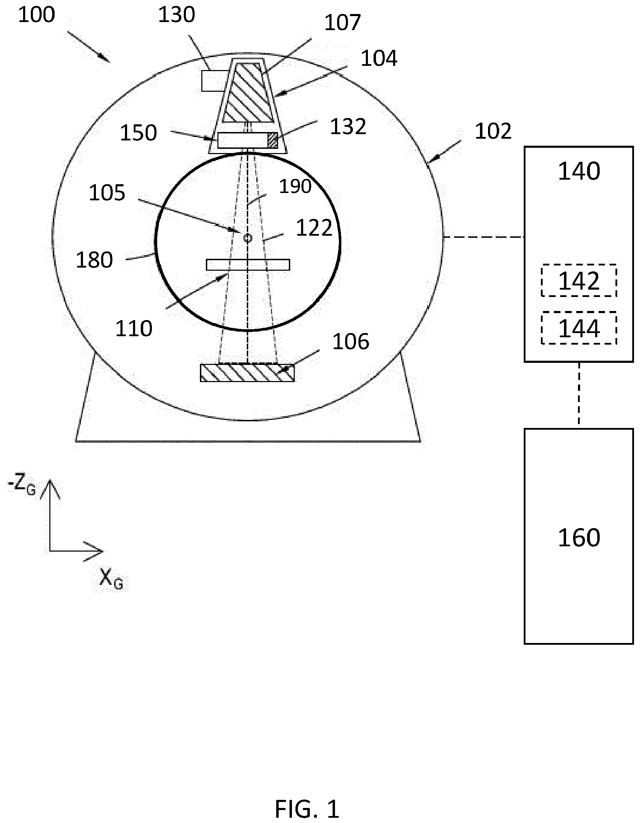

**Figure 1** depicts a radiotherapy apparatus according to the present disclosure, and particularly shows a cross-section through a radiotherapy apparatus 100 comprising a radiation head 104 and a beam receiving apparatus 106, both of which are attached to a gantry 102. The radiation head 104 includes a radiation source 107 which emits a beam of radiation 122. The radiation head 104 also includes a beam shaping apparatus 150 which controls the size and shape of the radiation field associated with the beam.

The beam receiving apparatus 106 is configured to receive radiation emitted from the radiation head 104, for the purpose of absorbing and/or measuring the beam of radiation. In the view shown in Figure 1, the radiation head 104 and the beam receiving apparatus 106 are positioned diametrically opposed to one another.

The gantry 102 is rotatable, and supports the radiation head 104 and the beam receiving apparatus 106 such that they are rotatable around an axis of rotation 105, which may coincide with the patient longitudinal axis. As shown in Figure 1, the gantry provides rotation of the radiation head 104 and the beam receiving apparatus 106 in a plane which is perpendicular to the patient longitudinal axis (e.g. a sagittal plane). Three gantry directions XG, YG, ZG can be defined, where the YG direction is perpendicular with gantry axis of rotation. The ZG direction extends from a point on the gantry corresponding to the radiation head, towards the axis of rotation of the gantry. Therefore, from the patient frame of reference, the ZG direction rotates around as the gantry rotates.

Figure 1 also shows a support surface 110 on which a subject (or patient) is supported during radiotherapy treatment. The radiation head 104 is configured to rotate around the axis of rotation 105 such that the radiation head 104 directs radiation towards the subject from various angles around the subject in order to spread out the radiation dose received by healthy tissue to a larger region of healthy tissue while building up a prescribed dose of radiation at a target region.

The radiotherapy apparatus 100 is configured to deliver a radiation beam towards a radiation isocentre which is substantially located on the axis of rotation 105 at the centre of the gantry 102 regardless of the angle at which the radiation head 104 is placed.

In the illustrated embodiment, the rotatable gantry 102 and radiation head 104 are dimensioned so as to allow a central bore 180 to exist. The central bore 180 provides opening sufficient to allow a subject to be positioned therethrough without the possibility of being incidentally contacted by the radiation head 104 or other mechanical components as the gantry rotates the radiation head 104 about the subject. In other embodiments, the rotatable gantry 102 may comprise a C-arm or other suitable apparatus that permits the placement of a subject beneath the radiation head 104 without risk of accidental collisions.

As shown in Figure 1, the radiation head 104 emits the radiation beam 122 along a beam axis 190 (or radiation axis or beam path), where the beam axis 190 is used to define the direction in which the radiation is emitted by the radiation head. The radiation beam 122 is incident on the beam receiving apparatus 106 which can include at least one of a beam stopper and a radiation detector. The beam receiving apparatus 106 is attached to the gantry 102 on a diametrically opposite side to the radiation head 104 to attenuate and/or detect a beam of radiation after the beam has passed through the subject.

The radiation beam axis 190 may be defined as, for example, a centre of the radiation beam 122 or a point of maximum intensity.

The beam shaping apparatus 150 delimits the spread of the radiation beam 122. The beam shaping apparatus 150 is configured to adjust the shape and/or size of a field of radiation produced by the radiation source. The beam shaping apparatus 150 does this by defining an aperture (also referred to as a window or an opening) of variable shape to collimate the radiation beam 122 to a chosen cross-sectional shape. In this example, the beam shaping apparatus 150 may be provided by a combination of a diaphragm and a multi-leaf collimator (MLC). Beam shaping apparatus 150 may also be referred to as a bean modifier.

The radiotherapy apparatus 100 may be configured to deliver both coplanar and non-coplanar (also referred to as tilted) modes of radiotherapy treatment. In coplanar treatment, radiation is emitted in a plane which is perpendicular to the axis of rotation of the radiation head 104. In non-coplanar treatment, radiation is emitted at an angle which is not perpendicular to the axis of rotation. In order to deliver coplanar and non-coplanar treatment, the radiation head 104 can move between at least two positions, one in which the radiation is emitted in a plane which is perpendicular to the axis of rotation (coplanar configuration) and one in which radiation is emitted in a plane which is not perpendicular to the axis of rotation (non-coplanar configuration).

In the coplanar configuration, the radiation head is positioned to rotate about a rotation axis and in a first plane. In the non-coplanar configuration, the radiation head is tilted with respect to the first plane such that a field of radiation produced by the radiation head is directed at an oblique angle relative to the first plane and the rotation axis. In the non-coplanar configuration, the radiation head is positioned to rotate in a respective second plane parallel to and displaced from the first plane. The radiation beam is emitted at an oblique angle with respect to the second plane, and therefore as the radiation head rotates the beam sweeps out a cone shape.

The beam receiving apparatus 106 remains in the same place relative to the rotatable gantry when the radiotherapy apparatus is in both the coplanar and non-coplanar modes. Therefore, the beam receiving apparatus 106 is configured to rotate about the rotation axis in the same plane in both coplanar and non-coplanar modes. This may be the same plane as the plane in which the radiation head rotates.

The beam shaping apparatus 150 is configured to reduce the spread of the field of radiation in the non-coplanar configuration in comparison to the coplanar configuration.

The radiation head 104 may be connected to a head actuator 130 which is configured to actuate the radiation head 104, for example between a coplanar configuration and one or more non-coplanar configurations. This may involve translation and rotation of the radiation head 104 relative to the gantry. In some implementations, the head actuator may include a curved rail along which the radiation head 104 may be moved to adjust the position and angle of the radiation head 104. The controller 140 may control the configuration of the radiation head 104 via the head actuator 130.

The beam shaping apparatus 150 includes a shaping actuator 132. The shaping actuator is configured to control the position of one or more elements in the beam shaping apparatus 150 in order to shape the radiation beam 122. In some implementations, the beam shaping apparatus 150 includes an MLC, and the shaping actuator 132 includes means for actuating leaves of the MLC. The beam shaping apparatus 150 may further comprise a diaphragm, and the shaping actuator 132 may include means for actuating blocks of the diaphragm. The controller 140 may control the beam shaping apparatus 150 via the shaping actuator 132.

The radiotherapy apparatus 100 includes a controller 140 which is programmed to control the radiation source 107, beam receiving apparatus 106 and the gantry 102. Controller 140 may perform functions or operations such as treatment planning, treatment execution, image acquisition, image processing, motion tracking, motion management, and/or other tasks involved in a radiotherapy process.

Controller 140 is programmed to control features of apparatus 100 according to a radiotherapy treatment plan for irradiating a target region, also referred to as a target tissue, of a patient. The treatment plan includes information about a particular dose to be applied to a target tissue, as well as other parameters such as beam angles, dose-histogram-volume information, the number of radiation beams to be used during therapy, the dose per beam, and the like. Controller 140 is programmed to control various components of apparatus 100, such as gantry 102, radiation head 104, beam receiving apparatus 106, and support surface 110, according to the treatment plan.

Hardware components of controller 140 may include one or more computers (e.g., general purpose computers, workstations, servers, terminals, portable/mobile devices, etc.); processors (e.g., central processing units (CPUs), graphics processing units (GPUs), microprocessors, digital signal processors (DSPs), field programmable gate arrays (FPGAs), special-purpose or specially-designed processors, etc.); memory/storage devices such as a memory (e.g., read-only memories (ROMs), random access memories (RAMs), flash memories, hard drives, optical disks, solid-state drives (SSDs), etc.); input devices (e.g., keyboards, mice, touch screens, mics, buttons, knobs, trackballs, levers, handles, joysticks, etc.); output devices (e.g., displays, printers, speakers, vibration devices, etc.); circuitries; printed circuit boards (PCBs); or other suitable hardware. Software components of controller 140 may include operation device software, application software, etc.

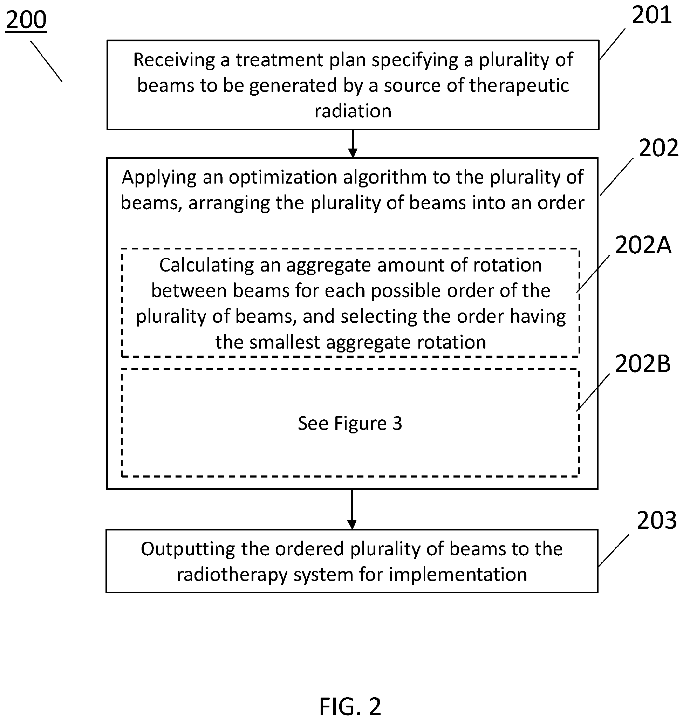

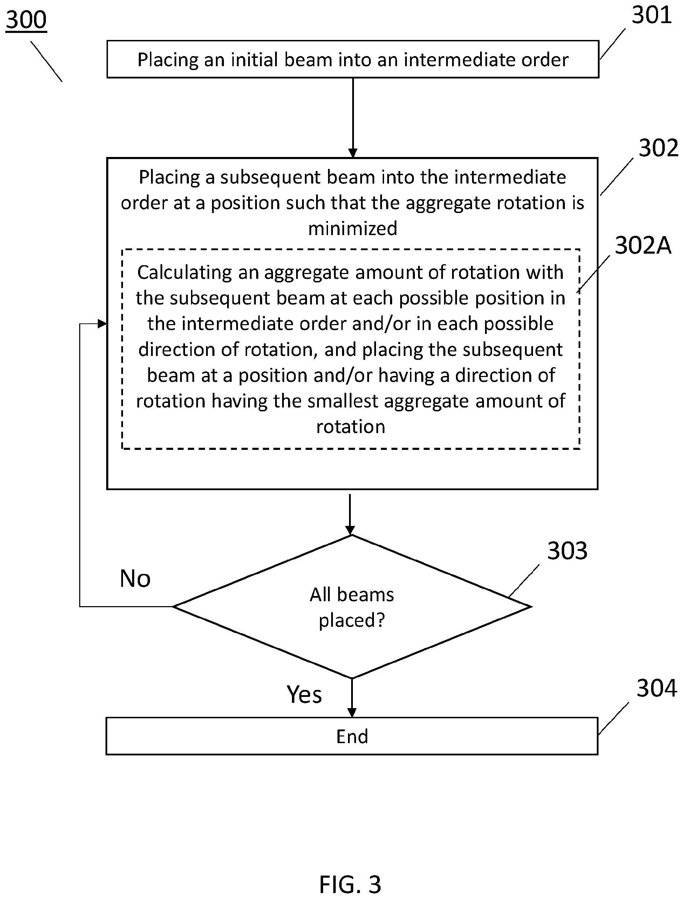

In the illustrated embodiment, the controller 140 comprises processing circuitry 142 and a non-transitory computer-readable storage medium. The processing circuitry 142 may comprise one or more processors (e.g., central processing units (CPUs), graphics processing units (GPUs), microprocessors, digital signal processors (DSPs), field programmable gate arrays (FPGAs), special-purpose or specially-designed processors, etc in any combination). The storage medium 144 may comprise memory (e.g., read-only memories (ROMs), random access memories (RAMs), flash memories, hard drives, optical disks, solid-state drives (SSDs), etc. The storage medium 144 may store instructions (e.g., code) which, when executed by the processing circuitry 142, cause the controller 140 to perform one or more methods as set out herein and described below, e.g., with respect to Figures 2 and 3.

In the illustrated embodiment, the apparatus 100 further comprises a treatment planning apparatus 160 communicatively coupled to the controller 140.

As noted above, a treatment plan defines one or more radiation beams and their associated parameters. For example, each radiation beam may be defined by one or more of the following parameters (among others):
- A start angle of the rotatable gantry, at which the radiation beam is initiated.
- A stop angle of the rotatable gantry, at which the radiation beam is halted.
- A cross-sectional shape of the radiation beam, as defined by one or more collimating elements, e.g., the beam-shaping apparatus 150.
- An energy of the radiation beam.

The respective start and stop angles for a beam may be the same (in which case the gantry does not rotate while the beam is active) or different (in which case the gantry rotates while the beam is active). In the latter case, rotation of the gantry from the start angle to the stop angle may be either clockwise or anticlockwise, and the direction of rotation may also be specified in the treatment plan.

The cross-sectional shape of the radiation beam may remain the same while the beam is initiated, or may vary as the gantry rotates from the start angle to the stop angle. Accordingly the treatment plan may specify how the cross-sectional shape changes as the gantry rotates. For example, the treatment plan may comprise information indicating a configuration of one or more elements of beam shaping apparatus 150, such as leaf configuration of an MLC of beam shaping apparatus 150, a configuration of a diaphragm of beam shaping apparatus 150, a configuration of an opening (e.g., window or aperture) of the MLC, and/or the like.

Radiotherapy systems may or may not permit the energy of radiation to be varied in different beams. Even if the energy of radiation is theoretically variable in a given system, the energy may nonetheless be set at a default value for each radiation beam. Accordingly the energy of a radiation beam may or may not be specified in the treatment plan.

The treatment-planning apparatus 160 generates the treatment plan in dependence on an image of the patient (e.g., the target area and its surrounding anatomy) as well as one or more clinical objectives provided by a clinician. The treatment plan is output to the controller 140 to be implemented by the radiotherapy apparatus.

Traditionally, for each patient, a treatment plan may be created using an optimization technique based on clinical and dosimetric objectives and constraints (e.g., the maximum, minimum, and mean doses to the tumour and critical organs). The treatment planning procedure may include using a three-dimensional (3D) image of the patient to identify a target region (e.g., the tumour) and to identify critical organs near the tumour. Creation of a treatment plan can be a time-consuming process where a planner tries to comply with various treatment objectives or constraints (e.g., dose volume histogram (DVH) objectives), taking into account their individual importance (e.g., weighting) in order to produce a treatment plan which is clinically acceptable. This task can be a time-consuming, trial-and-error process that is complicated by the various OARs, because as the number of OARs increases (e.g., 21 are commonly segmented in a head-and-neck treatment), so does the complexity of the process. OARs distant from a tumor may be easily spared from radiation, while OARs close to or overlapping a target tumor may be difficult to spare.

Those skilled in the art will therefore appreciate that the process of treatment planning is complex and involves the use of one or several optimization processes that seek to maximize the therapeutic dose delivered to the target area, while limiting the dose that is delivered to healthy tissue. Embodiments of the disclosure are not concerned with treatment planning per se, and thus further detail regarding the planning process itself is not provided herein. Typically, however, each treatment plan will define multiple beams, delivered from different angles of rotation of the gantry. Conventionally, these beams are delivered sequentially in the order defined by the treatment plan.

Embodiments of the disclosure improve upon conventional radiotherapy techniques by applying an optimization algorithm to the plurality of beams defined by the treatment plan, arranging the beams into an order such that an aggregate amount of rotation by the rotatable gantry between beams when delivering the treatment plan is minimized. By minimizing the amount of rotation required of the gantry when delivering a given treatment plan, the treatment plan can be delivered in less time. In this way, more patients can be treated per day than would otherwise be the case.

**Figure 2** is a flowchart of a method according to embodiments of the disclosure. The method may be implemented by a management apparatus for a radiotherapy system, such as the controller 140 and the apparatus 100 generally shown in Figure 1. In other embodiments, the management apparatus may not have any controlling functionality for the radiotherapy apparatus, but may instead be remote from the radiotherapy apparatus.

The method may be performed at or just prior to run-time of the treatment plan by the radiotherapy system. Alternatively, the method may be performed as part of or shortly after the treatment planning process

The method begins in step 201, in which the management apparatus receives, from a treatment planning apparatus (such as the apparatus 160), a treatment plan for delivery by the radiotherapy system. The treatment plan specifies a plurality of beams to be generated by the source of therapeutic radiation. Each beam is associated with first and second angles of the rotatable gantry, with the beam being initiated at one of the first and second angles (e.g., the start angle), and terminated at the other of the first and second angles (e.g., the stop angle). The first and second angles thus define the termini of the radiation beam; the radiation beam may be initiated at the first angle and terminated at the second angle, or initiated at the second angle and terminated at the first angle.

The first and second angles for at least one of the beams may be different from each other, such that that beam is generated and active while the gantry rotates between the first and second angles. In this way, the beam defines an arc. In some embodiments, the first and second angles for each of the plurality of beams are different from each other, such that each of the plurality of beams defines respective arcs. In other embodiments, the first and second angles for at least one of the beams may be the same as each other, such that the gantry does not rotate while that beam is generated and active (also known as a "stop and shoot" radiation beam).

In step 202, the management apparatus applies an optimization algorithm to the plurality of beams, which seeks to arrange the plurality of beams into an order such that an aggregate amount of rotation by the rotatable gantry between beams when delivering the treatment plan is minimized. That is, the optimization algorithm does not change the radiation beams themselves, but rather only optimizes the order in which those radiation beams are delivered to the patient. In some embodiments, the optimization algorithm also optimizes the direction of rotation of the gantry while a beam is active, e.g., when the first and second angles are different, whether the gantry rotates from the first angle to the second angle or vice versa.

Note that the amount of rotation of the gantry while the radiation beams are being delivered is fixed, as the radiation beams themselves are not affected by the optimization algorithm. Rather, the optimization algorithm seeks to minimize the total amount of rotation by the gantry between radiation beams, i.e., the amount of rotation by the gantry between the stop angle of the ith beam and the start angle of the (i+1)th beam, aggregated across the plurality of beams defined in the treatment plan.

In some embodiments, the optimization algorithm may additionally take into account the angle of the gantry at the start of a treatment session. For example, prior to a treatment session, the gantry may be rotated to a predefined "imaging" angle, at which an image is taken of the patient is taken for the purposes of correctly locating the patient within the radiotherapy system, and co-registering the patient's anatomy with the frame of reference of the radiotherapy system. The optimization algorithm may therefore seek to minimize the aggregate rotation of the gantry between delivery of radiation beams, with the constraint that the gantry begins at the predefined imaging angle.

In other embodiments, the gantry may be left at a final angle of rotation from a preceding treatment session (e.g., for a different patient). The optimization algorithm may therefore seek to minimize the aggregate rotation of the gantry between delivery of radiation beams, with the constraint that the gantry begins at the final angle of rotation from the preceding treatment session. This angle may be input by a user of the system, or stored in the management apparatus from the implementation of the preceding treatment session.

The optimization algorithm itself may take different forms.

In one embodiment, step 202 comprises the optional step 202A in which the management apparatus calculates an aggregate amount of rotation by the rotatable gantry between beams for each possible order of the plurality of beams, and selects the order having the smallest aggregate amount of rotation by the rotatable gantry between beams. Thus step 202A comprises a calculation of the aggregate rotations of the gantry when delivering the plurality of beams in all possible orders. Where there are n radiation beams and the direction of rotation of the gantry is not taken into account, there are n! possible orders to evaluate. Where the direction of rotation is also taken into account (i.e., where each radiation beam can be delivered in one of two directions of rotation), the number of possible orders increases by a factor of 2". For example, where there are three radiation beams, the management apparatus evaluates six possible orders if direction of rotation is not taken into account. If direction of rotation is taken into account, there are 48 possible orders to be evaluated.

Nonetheless, this complexity of processing may be feasible when the number of radiation beams is relatively low. Optionally, step 202A may be performed if the number of radiation beams is equal to or less than a threshold value.

If the number of radiation beams is relatively high, however, it may not be feasible to calculate the aggregate rotation for all possible orders. For example, where the treatment plan comprises 10 radiation beams, there are 3.6 x 10⁶ possible orders without considering different directions of rotation, and 3.7 x 10⁹ possible orders when considering different directions of rotation.

In other embodiments, therefore step 202 comprises the optional step 202B, which in turn points to the method 300 of Figure 3.

**Figure 3** is a flowchart of a method 300 of optimizing an order of radiation beams so as to minimize an aggregate amount of rotation between the beams.

The method begins in step 301, in which an initial beam is placed into an intermediate order. As used herein, the term "intermediate order" refers to the working sequence of radiation beams, as beams are added to it. The initial beam may be randomly selected from the plurality of beams defined by the treatment plan, or selected according to a predefined rule (e.g., the first beam defined by the treatment plan).

In step 302, a subsequent beam from the plurality of beams defined by the treatment plan is placed into the intermediate order at such a position that the aggregate rotation of the gantry when delivering the beams according to the intermediate order is minimized. The subsequent beam may be any of the beams defined by the treatment plan that is yet to be placed into the order. The subsequent beam may be randomly selected from the remaining beams defined by the treatment plan, or selected according to a predefined rule (e.g., the (i+1)th beam defined by the treatment plan, where the ith beam is the beam placed in a preceding iteration).

Let us consider a worked example. Assume that the initial beam is beam #1, and the subsequent beam, in a first iteration of the method, is beam #2. Beam #2 may be placed before or after beam #1, and therefore the possible intermediate orders in step 302 are:
#1, #2
#2, #1

If the method is considering each possible direction of rotation, there are four possible orders after the first iteration:
Possible order 1: #1, #2
Possible order 2: #1, - #2
Possible order 3: #2, #1
Possible order 4: - #2, #1
where a "=` sign indicates the reverse direction of rotation relative to a beam without the "=` sign. Note that this example does not consider different directions of rotation for the initial beam, beam #1.

Step 302 may include the optional step 302A whereby the aggregate amount of rotation is calculated with the subsequent radiation beam at each possible position in the intermediate order and, optionally, in each possible direction of rotation. The subsequent beam is placed at the order and/or with the direction of rotation having the least aggregate rotation of the gantry.

Consider again the example above, taking into account different directions of rotation. According to step 302A, the management apparatus calculates the amount of rotation of the gantry in moving from the end angle of the first beam in the order, to the start angle of the second beam in the order. In more detail:
Possible order 1: rotation from second angle of beam #1 to first angle of beam #2
Possible order 2: rotation from second angle of beam #1 to second angle of beam #2
Possible order 3: rotation from second angle of beam #2 to first angle of beam #1
Possible order 4: rotation from first angle of beam #2 to first angle of beam #1

Whichever of these four angles is the least is selected as the intermediate order after the first iteration. Thus, in this first iteration, the management apparatus considers four different orders, and selects the order (which at this stage is an intermediate order) having the least rotation.

In step 303, it is determined whether all of the beams defined by the treatment plan have been placed into the order. If all of the beams have been placed, the process is at an end in step 304 and the order of the beams has been optimized. The final "intermediate" order becomes the optimized order of radiation beams.

If not all of the beams have been placed, the process reverts to step 302 and another one of the remaining beams is placed into the intermediate order. At each iteration of the method, another beam is placed into the order until all of the beams have been placed.

Note that the number of calculations at each iteration of the method 300 is significantly fewer than calculating all possible orders. For example, in a first iteration of the method (placing a second beam into the order) and considering different directions of rotation, there are four possible orders to be calculated. In a second iteration of the method (placing a third beam into the order) and considering different directions of rotation, there are six possible orders to be calculated: three different positions, and two different directions of rotation. For a treatment plan defining three radiation beams, there are 10 calculations in total. For a treatment plan defining ten radiation beams, there are 108 calculations in total. The computational complexity of the search is greatly reduced.

The method 300 may be extended to take into account a constraint that the gantry starts from a predefined angle of rotation, e.g., a predefined angle from which images are acquired, or a final angle of rotation from a preceding treatment session. For example, the predefined angle may be included as a "beam" that always appears at the start of the order.

By calculating the aggregate amount of rotation for every possible order of radiation beams, step 202A enables the global minimum amount of rotation to be found, and selected for implementation by the radiotherapy apparatus. This may be preferable for treatment plans with relatively few radiation beams. The method set out with respect to step 202B and Figure 3 may not be guaranteed to find the global minimum amount of rotation. However, it will return a local minimum amount of rotation, and significantly reduces the computational complexity involved. This optimization algorithm may be preferable for treatment plans with a relatively high number of radiation beams.

Returning to Figure 2, and in step 203 the management apparatus outputs the ordered plurality of beams to the radiotherapy system for implementation. The amount of time taken to deliver the treatment plan is reduced as a result of the re-ordered radiation beams.

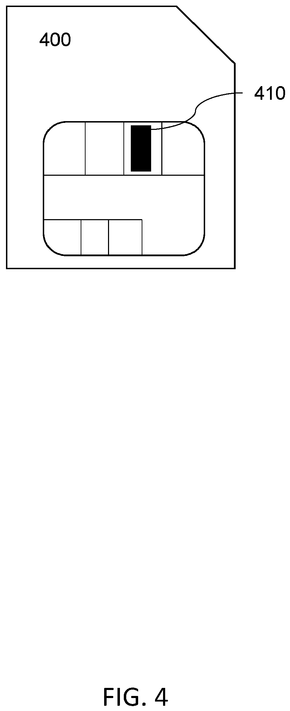

**Figure 4** is a schematic illustrating a computer program product 400 according to some embodiments. The various methods described above may be implemented by a computer program. The computer program may include computer code (e.g. instructions) 410 arranged to instruct a computer to perform the method 200 described with respect to Figure 2 and/or the method 300 described with respect to Figure 3.

For example, the steps of the methods described in relation to Figure 2 may be performed by the computer code 410. The steps of the methods described above may be performed in any suitable order. The computer program and/or the code 410 for performing such methods may be provided to an apparatus, such as a computer, on one or more computer readable media or, more generally, a computer program product 400)), depicted in Figure 4. The computer readable media may be transitory or non-transitory. The one or more computer readable media 400 could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD. The instructions 410 may also reside, completely or at least partially, within the memory 142.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may comprise a special-purpose processor, such as an FPGA or an ASIC. A hardware component may also include programmable logic or circuitry that is temporarily configured by software to perform certain operations.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

Unless specifically stated otherwise, as apparent from the following discussion, it is appreciated that throughout the description, discussions utilizing terms such as " receiving", "determining", "comparing", "enabling", "maintaining," "identifying,", "obtaining", "accessing" or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

While certain embodiments have been described, these embodiments have been presented by way of example only, and are not intended to limit the scope of the claims appended hereto. Indeed, the novel methods and apparatus described herein may be embodied in a variety of other forms; furthermore, various omissions, substitutions and changes in the form of methods and apparatus described herein may be made.

## Claims

1. A method performed by a management apparatus for a radiotherapy system, the radiotherapy system comprising a source of therapeutic radiation mounted on a rotatable gantry, the method comprising:
receiving, from a treatment planning apparatus, a treatment plan for delivery by the radiotherapy system, the treatment plan specifying a plurality of beams to be generated by the source of therapeutic radiation, wherein each beam is associated with first and second angles of the rotatable gantry, wherein the beam is initiated at one of the first and second angles, and terminated at the other of the first and second angles;
applying an optimization algorithm to the plurality of beams, the optimization algorithm arranging the plurality of beams into an order such that an aggregate amount of rotation by the rotatable gantry between beams when delivering the treatment plan is minimized; and
outputting the ordered plurality of beams to the radiotherapy system for implementation.

2. The method according to claim 1, wherein the optimization algorithm further specifies a direction of rotation between the first and second angles for each beam.

3. The method according to claim 1 or 2, wherein the plurality of beams is arranged into an order in which an aggregate amount of rotation by the rotatable gantry between beams when delivering the treatment plan is reduced, taking into account a final angle of rotation of the rotatable gantry from an immediately preceding treatment plan delivered by the radiotherapy apparatus.

4. The method according to any one of the preceding claims, wherein the optimization algorithm comprises:
a. placing an initial beam from the plurality of beams into an intermediate order;
b. placing a subsequent beam from a remainder of the plurality of beams into the intermediate order, wherein the subsequent beam is placed at a position in the intermediate order such that the aggregate amount of rotation by the rotatable gantry between beams in the intermediate order is minimized; and
c. repeating step b until the plurality of beams are arranged into the order.

5. The method according to claim 4, wherein step b comprises calculating aggregate amounts of rotation by the rotatable gantry with the subsequent beam at each possible position in the intermediate order, and placing the subsequent beam at a position in the intermediate order having the smallest aggregate amount of rotation of the rotatable gantry between beams according to the intermediate order.

6. The method according to claim 4 or 5, wherein step b comprises calculating aggregate amounts of rotation by the rotatable gantry with the subsequent beam placed at each possible position in the intermediate order and in each direction between the first and second angles, and placing the subsequent beam at a position in the intermediate order and in a direction of rotation having the smallest aggregate amount of rotation of the rotatable gantry between beams according to the intermediate order.

7. The method according to any one of claims 4 to 6 as dependent on claim 2, wherein step b comprises placing a subsequent beam from a remainder of the plurality of beams into the intermediate order such that the aggregate amount of rotation by the rotatable gantry between beams in the intermediate order is minimized, taking into account a final angle of rotation of the rotatable gantry from an immediately preceding treatment session delivered by the radiotherapy apparatus.

8. The method according to any one of claims 4 to 6, wherein step b comprises placing a subsequent beam from a remainder of the plurality of beams into the intermediate order such that the aggregate amount of rotation by the rotatable gantry between beams in the intermediate order is minimized, taking into account an imaging position of the rotatable gantry.

9. The method according to any one of claims 1 to 3, wherein the optimization algorithm comprises calculating an aggregate amount of rotation by the rotatable gantry between beams for each possible order of the plurality of beams, and selecting the order having the smallest aggregate amount of rotation by the rotatable gantry between beams.

10. The method according to any one of the preceding claims, wherein the first angle for a first beam of the plurality of beams is different to the second angle for the first beam.

11. The method according to claim 10, wherein the respective first angle for each beam of the plurality of beams is different to the respective second angle

12. The method according to any one of claims 1 to 10, wherein the first angle for a second beam of the plurality of beams is the same as the second angle for the second beam.

13. The method according to any one of the preceding claims, wherein the method is performed at run-time of the treatment plan by the radiotherapy system.

14. The method according to any one of the preceding claims, wherein the treatment planning apparatus is co-located with the management apparatus.

15. A management apparatus for a radiotherapy system, comprising processing circuitry and a non-transitory computer-readable medium storing instructions which, when executed by the processing circuitry, cause the management apparatus to:
receive, from a treatment planning apparatus, a treatment plan for delivery by a radiotherapy system comprising a source of therapeutic radiation mounted on a rotatable gantry, the treatment plan specifying a plurality of beams to be generated by the source of therapeutic radiation, wherein each beam is associated with first and second angles of the rotatable gantry, wherein the beam is initiated at one of the first and second angles, and terminated at the other of the first and second angles;
apply an optimization algorithm to the plurality of beams, the optimization algorithm arranging the plurality of beams into an order such that an aggregate amount of rotation by the rotatable gantry between beams when delivering the treatment plan is minimized; and
output the ordered plurality of beams to the radiotherapy system for implementation.

16. A non-transitory computer-readable medium storing instructions which, when executed by processing circuitry, cause the processing circuitry to:
receive, from a treatment planning apparatus, a treatment plan for delivery by a radiotherapy system comprising a source of therapeutic radiation mounted on a rotatable gantry, the treatment plan specifying a plurality of beams to be generated by the source of therapeutic radiation, wherein each beam is associated with first and second angles of the rotatable gantry, wherein the beam is initiated at one of the first and second angles, and terminated at the other of the first and second angles;
apply an optimization algorithm to the plurality of beams, the optimization algorithm arranging the plurality of beams into an order such that an aggregate amount of rotation by the rotatable gantry between beams when delivering the treatment plan is minimized; and
output the ordered plurality of beams to the radiotherapy system for implementation.
